Europäisches Patentamt

European Patent Office

Office européen des brevets

(19)

(11) Veröffentlichungsnummer: **0 178 371**
A2

(12) # EUROPÄISCHE PATENTANMELDUNG

(21) Anmeldenummer: 85102858.9

(22) Anmeldetag: 13.03.85

(51) Int. Cl.⁴: **A 61 M 5/14**

(30) Priorität: 05.06.84 DE 3420893

(43) Veröffentlichungstag der Anmeldung:
23.04.86 Patentblatt 86/17

(84) Benannte Vertragsstaaten:
AT BE CH DE FR GB IT LI LU NL SE

(71) Anmelder: INTERMEDICAT GMBH
Gerliswilstrasse 74
CH-6020 Emmenbrücke(CH)

(72) Erfinder: Köhler, Uwe, Dipl.-Phys
Elisabethweg 12
D-3588 Homberg(DE)

(72) Erfinder: Stöber, Herbert
Über dem Rötter 1
D-3513 Staufenberg 1(DE)

(74) Vertreter: von Kreisler, Alek, Dipl.-Chem. et al,
Deichmannhaus am Hauptbahnhof
D-5000 Köln 1(DE).

(54) Dosiervorrichtung für die subkutane und intravenöse Zuführung flüssiger Arzneimittel.

(57) Die Dosiervorrichtung weist ein Gehäuse (10) auf, das am Körper eines Patienten befestigt werden kann. Im Gehäuse (10) befindet sich eine flexible Hülle (17), die ein flüssiges Injektat enthält, das durch Drehen des Deckels 14 in einen zu einer Verweilkanüle führenden Schlauch (23) ausgeschoben wird. Die Dosiervorrichtung erlaubt das intermittierende Injizieren eines Medikaments durch den Patienten selbst, ohne daß die Verbindung von dem Reservoir zum Patienten zwischendurch unterbrochen werden muß.

FIG.1

VON KREISLER   SCHÖNWALD   EISHOLD   FUES
VON KREISLER   KELLER   SELTING   WERNER

0178371

PATENTANWÄLTE

Dr.-Ing. von Kreisler † 1973
Dr.-Ing. K. W. Eishold † 1981
Dr.-Ing. K. Schönwald
Dr. J. F. Fues
Dipl.-Chem. Alek von Kreisler
Dipl.-Chem. Carola Keller
Dipl.-Ing. G. Selting
Dr. H.-K. Werner

Intermedicat GmbH
Gerliswilstraße 74

CH - 6020 Emmenbrücke
Schweiz

BEZEICHNUNG GEÄNDERT
siehe Titelseite

DEICHMANNHAUS AM HAUPTBAHNHOF
D-5000 KÖLN 1
12. März 1985
Sg/Fe

Dosiervorrichtung für Injektabilien

Die Erfindung betrifft eine Dosiervorrichtung für Injektabilien, mit einem Behälter, dessen Volumen durch eine bewegbare Ausstoßvorrichtung veränderbar ist.

Zur Vermeidung häufiger Injektionen bei intermittierenden Medikamentengaben (Insuline, Heparin) ist es bekannt, eine Verweilkanüle zu verwenden, die nach subkutaner Plazierung an eine Spritze oder über eine Schlauchleitung an einen Medikamentenbehälter angeschlossen wird und anschließend wieder abgetrennt werden kann, um bis zur nächsten Injektion am Körper zu verbleiben. Das ständige An- und Abkuppeln einer Spritze oder einer Schlauchleitung an die Verweilkanüle kann vom Patienten nur umständlich durchgeführt werden, wenn die Verweilkanüle im Arm des Patienten steckt, weil der Patient dann nur eine Hand zur Verfügung hat. Es ist dann stets die Hilfe einer anderen Person erforderlich. Wenn bei jeder Injektion eine neue Spritze benutzt wird, stellt dies einen erheblichen Kostenaufwand dar, weil jede Spritze nach dem Ausschieben des Injektats fortgeworfen wird. Ein weiterer Nachteil der bekannten Systeme besteht darin, daß durch das ständige an- und abkuppeln an die Verweilkanüle Kontaminationen in das System gelangen können.

Der Erfindung liegt die Aufgabe zugrunde, eine Dosiervorrichtung der eingangs genannten Art zu schaffen, die am Körper des Patienten getragen werden kann und es dem Patienten ermöglicht, die Injektion durchzuführen, ohne daß jedesmal ein Behälter oder eine Spritze an die Verweilkanüle angekuppelt werden muß.

Die Lösung dieser Aufgabe besteht erfindungsgemäß darin, daß der Behälter eine mit einer flexiblen Leitung verbundene geschlossene verformbare Hülle ist, die in einem starren Gehäuse untergebracht ist, daß die Ausstoßvorrichtung aus einem am Gehäuse abgestützten Vortriebsteil besteht, das gegen die Hülle drückt und relativ zu dem Gehäuse bewegbar ist, und daß die Leitung durch eine Öffnung des Gehäuses hindurchragt.

Bei der erfindungsgemäßen Dosiervorrichtung ist das Injektat in einer geschlossenen Hülle enthalten, aus der es unter Verformung der Hülle herausgedrückt wird. Das Injektat kommt mit dem Behälter, der gewissermaßen eine Schutzkapsel darstellt, überhaupt nicht in Berührung. Durch Bewegen des Vortriebsteils des Behälters wird das Injektat aus der Hülle herausgedrückt. Das Injektat wird durch die flexible Leitung zu einer Verweilkanüle geführt. Bei jeder Injektion wird das Vortriebsteil ein Stück weiter in das Gehäuse hineinbewegt. Dieses Gehäuse kann mit Klebebändern, Klettenbändern oder anderen Befestigungsvorrichtungen leicht Deckels entspricht. Hierbei erfolgt ein kontrolliertes Zusammenfalten der umfangsmäßigen Faltenwand der Hülle und die Linearität zwischen Vorschubbewegung und ausgeschobener Injektatmenge wird verbessert.

Das Gerät kann auch zum Ansaugen des Injektats aus einem Medikamentenbehältnis (Glasfläschchen) benutzt werden. In diesem Falle wird der zusammengequetschte Faltenbalg über die Verbindungsleitung mit der in dem Glasfläschchen liegenden Kanüle verbunden. Durch Rückdrehen der Vortriebsscheibe wird der Faltenberg entspannt und kann, durch seine Eigenelastizität bedingt, Flüssigkeit in seinen Innenraum saugen.

Während das Gehäuse wiederbenutzt werden kann, bildet die Hülle ein Wegwerfteil, das in das Gehäuse eingesetzt wird, nachdem der Deckel abgenommen worden ist. Um das Einführen der Hülle in das Gehäuse zu erleichtern, erstreckt sich von der Öffnung ein Schlitz bis zum deckelseitigen Rand des Gehäuses. Dieser Schlitz dient einerseits dem Einführen der flexiblen Leitung und er bildet andererseits auch eine Anzeigevorrichtung zum Erkennen der Position des Vortriebsteils, so daß der Benutzer durch Beobachtung der Position des Vortriebsteils in dem Schlitz weiß, wann die Hülle leer sein wird.

An dem Vortriebsteil und dem Gehäuse können Markierungen für die Anzeige der Drehstellung des Vortriebsteils vorgesehen sein, damit bei jeder Injektion die vorgeschriebene Dosierung erfolgen kann. Hierbei können entweder an dem Gehäuse eine Skala und an dem Deckel eine Markierung, z.B. ein Zeiger, angebracht sein, oder die Skala kann sich am Deckel und der Zeiger am Gehäuse befinden.

am Körper des Patienten befestigt werden. Wenn das Gehäuse auf diese Weise fixiert ist, kann das Bewegen des Vortriebsteils mit einer Hand erfolgen.

Vorzugsweise ist das Vortriebsteil eine über Gewinde mit dem Gehäuse in Eingriff stehende Scheibe, die durch Drehen relativ zu dem Gehäuse bewegbar ist. Durch Drehen der Scheibe wird diese in das Gehäuse hineinbewegt, wodurch die Hülle zunehmend zusammengedrückt wird, um das Injektat aus der Hülle auszustoßen. Durch Kontrolle des Drehwinkels, um den die Scheibe verdreht wird, kann die Menge des aus der Hülle ausgestoßenen Injektats dosiert werden. Die Menge, die bei einer Drehung der Scheibe um einen vorgegebenen Winkel ausgestoßen wird, sollte über den gesamten Verschiebebereich der Scheibe konstant sein. Um dies zu erreichen, weist die Hülle zwei parallele Wände auf, zwischen denen sich eine umlaufende Faltenwand erstreckt. Die Hülle hat dabei annähernd die Form eines Zylinders, dessen Länge durch die Bewegung des Vortriebsteils verändert wird.

Gemäß einer bevorzugten Weiterbildung der Erfindung weist die Innenseite des Deckels einen in Richtung auf die Hülle vorstehenden koaxialen Vorsprung auf, der von einem Ringkanal zur Aufnahme des Randes der Hülle umgeben ist. Die obere Wand der Hülle wird von dem glatten Vorsprung der Scheibe flach gehalten, während der Rand der Hülle ziehharmonikaartig zusammengefaltet wird. Dadurch wird eine weitgehend lineare Funktion zwischen Deckelbewegung und Injektatmenge erreicht.

Vorteilhafterweise ist die Unterseite der Hülle durch ein starres Stützteil abgestützt, dessen Profil im wesentlichen einem Negativ des Profils der Innenseite des Grundsätzlich ist es möglich, an dem Vortriebsteil einen Handgriff zum Bewegen dieses Vortriebsteils vorzusehen. Hierbei würde aber die Gefahr bestehen, daß das Vortriebsteil unbeabsichtigt verstellt wird. Gemäß einer vorteilhaften Weiterbildung der Erfindung .weist das Vortriebsteil einen Schlitz zum Einsetzen eines Drehwerkzeugs auf. Ein solches Drehwerkzeug kann ein Schraubenzieher, eine Münze o.dgl. sein.

Die erfindungsgemäße Dosiervorrichtung, die am menschlichen Körper zu tragen ist, sollte gesichert werden können, so daß infolge von Bewegungen des Körpers oder durch Schwerkrafteinfluß Injektat nicht unkontrolliert aus dem Behälter ausfließen kann. Um dies zu erreichen, wird gemäß einer bevorzugten Weiterbildung der Erfindung vorgeschlagen, daß an dem Gehäuse eine Klemmvorrichtung zum Zusammenquetschen eines Abschnitts der festen Leitung vorgesehen ist und daß die Klemmvorrichtung ein Betätigungsorgan aufweist, das in einer Verriegelungsstellung das Verstellen des Vortriebsteils verhindert und gleichzeitig die flexible Leitung abquetscht und in einer Freigabestellung das Vortriebsteil und die flexible Leitung freigibt. Zur Durchführung der Injektion wird das Betätigungsorgan in die Freigabestellung gebracht, in der es möglich ist, das Vortriebsteil zu bewegen. Dabei wird die Klemmung der flexiblen Leitung aufgehoben. Anschließend wird das Betätigungsorgan wieder in die Verriegelungsstellung gebracht, wodurch das Vortriebsteil gegen Bewegungen gesichert und die flexible Leitung verschlossen wird.

Im folgenden werden unter Bezugnahme auf die Zeichnungen Ausführungsbeispiele der Erfindung näher erläutert.

Es zeigen:

Fig. 1    einen Längsschnitt durch eine erste Ausführungsform der Dosiervorrichtung,

Fig. 2    eine Draufsicht der Dosiervorrichtung nach
          Fig. 1,

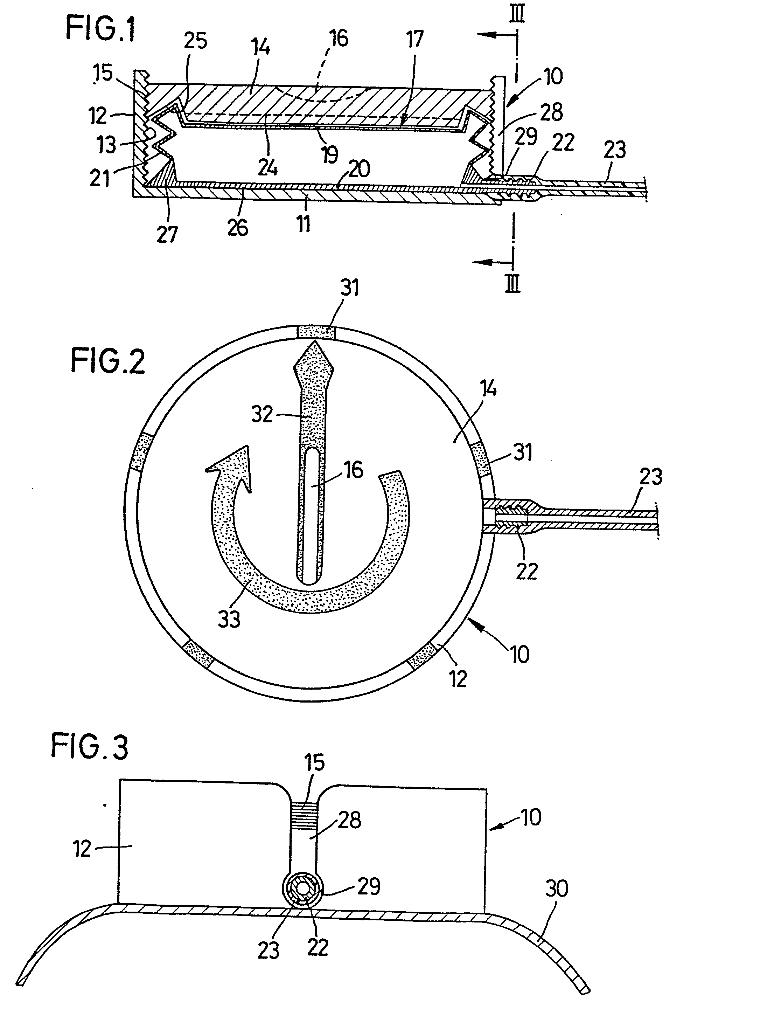

Fig. 3    einen Schnitt entlang der Linie III-III von
          Fig. 1,

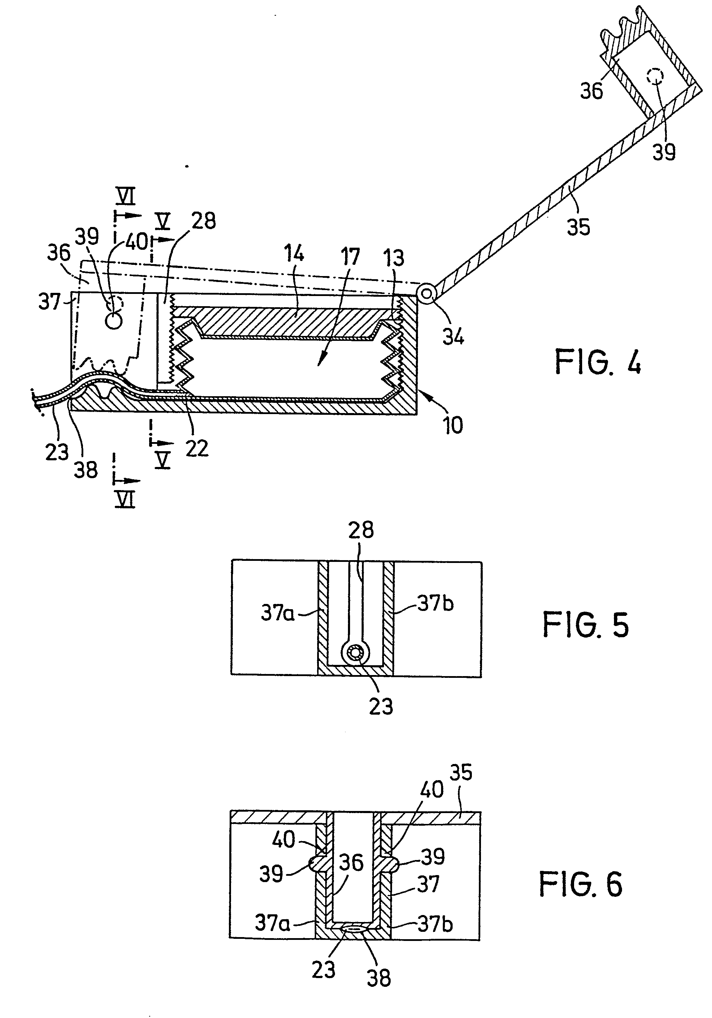

Fig. 4    einen Längsschnitt durch eine zweite Ausführungsform der Dosiervorrichtung,

Fig. 5    einen Schnitt entlang der Linie V-V von Fig.
          4, jedoch ohne Darstellung des Deckels,

Fig. 6    einen Schnitt entlang der Linie VI-VI von
          Fig. 4,

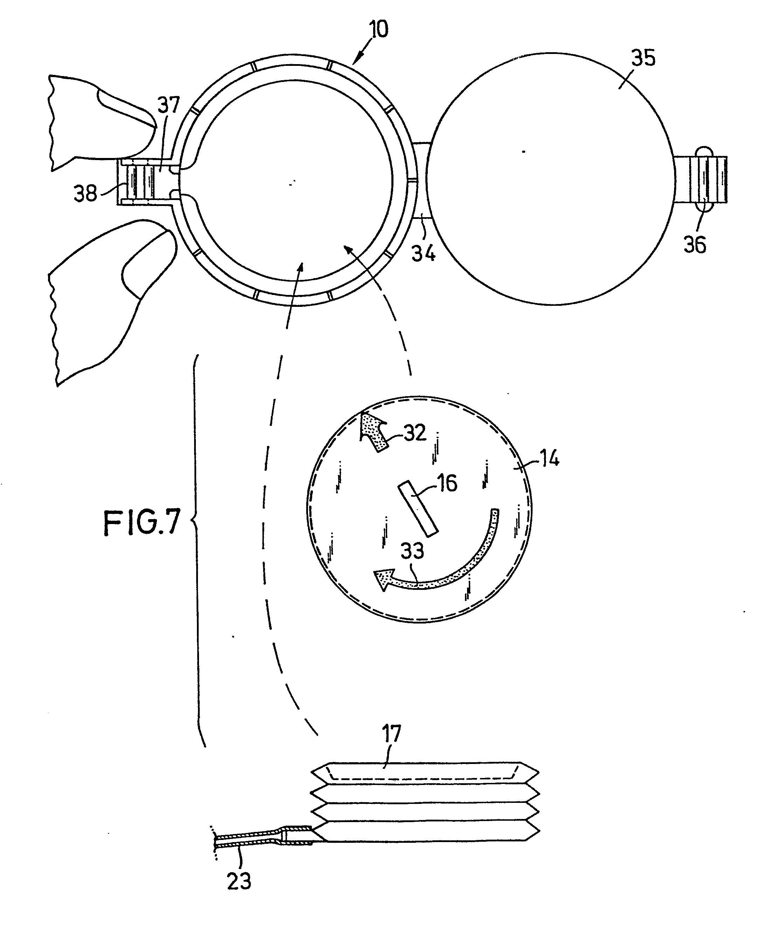

Fig. 7    eine Explosionsdarstellung der Dosiervorrichtung nach Fign. 4 bis 6,

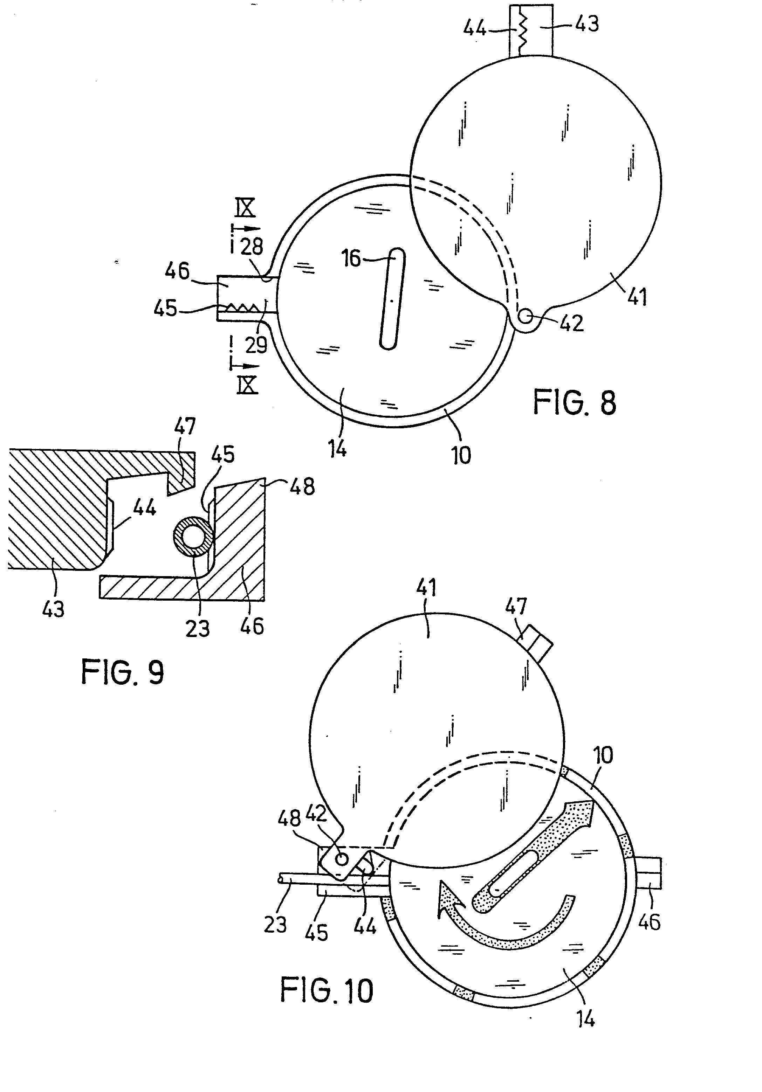

Fig. 8    eine Draufsicht eines dritten Ausführungsbeispiels,

Fig. 9    einen Schnitt entlang der Linie IX-IX von
          Fig. 8 kurz vor dem Einrasten des Deckels und

Fig. 10   eine Draufsicht auf ein drittes Ausführungsbeispiel der Dosiervorrichtung.

Die Dosiervorrichtung nach Fign. 1 bis 3 weist ein starres zylindrisches Gehäuse 10 mit einer Bodenwand 11 und einer umlaufenden Seitenwand 12 auf. An der Innenseite der Seitenwand 12 ist ein Innengewinde 13 angebracht. Die Oberseite des Gehäuses 10 ist offen und kann durch die Vortriebsscheibe 14 verschlossen werden. Die Vortriebsscheibe 14 weist an ihrem Umfang ein Außengewinde 15 auf, das in das Innengewinde 13 des Gehäuses 10 eingeschraubt wird. In der Mitte der Vortriebsscheibe 14 befindet sich ein Schlitz 16, in den ein Drehwerkzeug, z.B. eine Münze, hineingesteckt werden kann, um die Vortriebsscheibe 14 zu drehen.

Im Innern des Gehäuses 10 befindet sich die herausnehmbare Hülle 17, die aus einem flexiblen Material besteht und allseitig geschlossen ist. Die Hülle 17 dient zur Aufnahme des flüssigen Injektats 18. Sie weist zwei parallele Wände 19 und 20 auf, zwischen denen sich die umlaufende Faltenwand 21 erstreckt. Von dem der unteren Wand 20 benachbarten Ende der Faltenwand 21 steht ein rohrförmiges Anschlußstück 22 radial ab. Dieses Anschlußstück 22 ist an seiner Außenseite mit einer Riffelung versehen. Auf das Anschlußstück 22 kann ein Schlauch 23 aufgeschoben werden, der zu der (nicht dargestellten) Verweilkanüle führt. Das Anschlußstück 22 ist der Hülle 17 fest angeformt.

An der Unterseite der Vortriebsscheibe 14 befindet sich ein Vorsprung 24 mit ebener Unterseite. Dieser Vorsprung 24 ist von einem Ringkanal 25 umgeben, der sich bis zu dem äußeren Rand der Vortriebsscheibe 14 erstreckt. Der Ringkanal 25 dient zur Aufnahme der Faltenwand 21, wenn die Vortriebsscheibe 14 sich in der Endstellung im voll eingeschraubten Zustand befindet. Die der Bodenwand 11 des Gehäuses zugewandte Wand 20 der Hülle 17 ist von einer ebenen Platte 26 abgestützt, die an der Bodenwand 11 anliegt und einen aufragenden Rand 27 hat, dessen Kontur annähernd derjenigen des Ringkanals 25 angepaßt ist, so daß die Faltenwand 21 im zusammengefalteten Zustand zwischen dem Rand 27 und dem Ringkanal 25 zusammengedrückt wird, um das zwischen den Falten befindliche Injektat auszudrücken.

Die Gestaltung der Falten der Hülle 17 (Faltenlänge und Faltentiefe) ist im Verhältnis zum Durchmesser der Hülle 17 so bemessen, daß die Ungenauigkeit der Volumenänderung pro komprimierter Längeneinheit bei der Vortriebsbewegung des Deckels einen geforderten Wert (ca. 3%) nicht überschreiten kann.

In der Umfangswand des Gehäuses 10 ist ein Schlitz 28 vorgesehen, der in eine runde Öffnung 29, die sich unmittelbar über der Bodenwand 11 befindet, mündet. Die Breite des Schlitzes 28 ist kleiner als der Durchmesser der Öffnung 29. Der Schlitz 28 dient zum Einführen des Anschlußstückes 22 beim Einsetzen der gefüllten Hülle 17 in das Gehäuse 10. Dabei kann das Ende des Schlauchs 23 auf den aus dem Gehäuse 10 hinausragenden Bereich des Anschlußstückes 22 aufgeschoben sein. Wenn das Anschlußstück 22 sich in der Öffnung 29 befindet, wird das Schlauchende ein Stück in die Öffnung 29 hineingezogen, so daß das Anschlußstück 22 mit aufgeschobenem Schlauchende nicht mehr durch den Schlitz 28 geschoben werden kann. Wie Fig. 3 zeigt, ist das Gewinde 15 des Deckels 14 durch den Schlitz 28 hindurch sichtbar, so daß eine Grobkontrolle der Position der Vortriebsscheibe erfolgen kann.

An der Bodenwand 11 des Gehäuses 10 ist ein Befestigungsband 30 angebracht, um das Gehäuse am Körper des Patienten befestigen zu können.

An der Stirnseite der Umfangswand 12 des Gehäuses 10 befinden sich Markierungen 31, die in gleichmäßigen Winkelabständen verteilt angeordnet sind. An der Vortriebsscheibe 14 ist ein radialer Zeiger 32 fest angebracht. Bei der Drehung der Vortriebsscheibe 14 bewegt sich die Spitze des Zeigers von einer Markierung 31 zur nächsten. Der Drehsinn ist durch einen aufgedruckten Pfeil 33 markiert. Erforderlichenfalls kann eine Rückdrehsicherung vorgesehen sein, die verhindert, daß die Vortriebsscheibe in Gegenrichtung gedreht werden kann.

Bei Benutzung der Dosiervorrichtung wird eine mit Injektat 18 gefüllte Hülle 17 in das leere Gehäuse 10 eingesetzt. Dann wird die Vortriebsscheibe 14 aufgeschraubt. Die in dem Gehäuse enthaltene Luft entweicht zwischen den Gewinden 13 und 15 oder durch eine (nicht dargestellte) Entlüftungsöffnung in der Vortriebsscheibe. Die Oberseite der Hülle 17 hat eine Kontur, die der Kontur der Unterseite der Vortriebsscheibe 14 im wesentlichen angepaßt ist. Durch Drehen der Vortriebsscheibe 14 in Richtung des Pfeiles 33 dringt die Vortriebsscheibe 14 in das Gehäuse 10 ein, wodurch die flexible Hülle 17 zusammengedrückt und Injektat 18 in den Schlauch 23 hinein ausgestoßen wird. Ein Injektionsvorgang wird ausgeführt, indem in den Schlitz 16 ein Drehwerkzeug, z.B. eine Münze, eingesteckt und die Vortriebsscheibe 14 von einer Markierung 31 bis zur nächsten Markierung gedreht wird. Dadurch wird eine vorbestimmte Menge an Injektat durch den Schlauch 23 und die Verweilkanüle in den Körper des Patienten injiziert. Wenn die Hülle 17 leer ist, wird die Vortriebsscheibe 14 abgeschraubt und die Hülle 17 durch eine neue gefüllte Hülle ersetzt.

Das Ausführungsbeispiel der Fign. 4 bis 7 gleicht weitgehend dem ersten Ausführungsbeispiel, so daß eine detaillierte nochmalige Erläuterung derjenigen Teile, die bei beiden Ausführungsbeispielen einander gleich sind, unterbleibt. An dem oberen Rand des Gehäuses 10 ist über eine Gelenkachse 34 ein Betätigungsorgan 35 in Form eines Deckels angebracht, der in der Schließstellung die obere Öffnung des Gehäuses 10 verschließt und die Vortriebsscheibe 14 vollständig bedeckt. Die Gelenkachse 34 verläuft horizontal und somit rechtwinklig zur Vortriebsrichtung der Vortriebsscheibe 14. An dem der Gelenkachse 34 abgewandten Ende des Betätigungsorgans 35 befindet sich ein radial abstehender Ansatz, der als Klemmorgan 36 ausgebildet ist. Ein entsprechender Ansatz 37, der einen Freiraum zum Eintauchen des Klemmorgans 36 aufweist, steht von dem Gehäuse 10 ab. Der Freiraum des Ansatzes 37 des Gehäuses 10 wird von zwei vertikalen Wänden 37a und 37b begrenzt, die durch eine Bodenwand miteinander verbunden sind. Die Bodenwand bildet das zweite Klemmorgan 38. Der vertikale Schlitz 28 zum Einführen des Schlauchs 23 mündet in den Freiraum zwischen den Wänden 37a und 37b. Der Schlauch 23 liegt normalerweise lose auf der profilierten Fläche des zweiten Klemmorgans 38 auf. Wenn der Deckel 35 geschlossen wird und die Vortriebsscheibe 14 überdeckt, taucht das Klemmorgan 36 des Deckels 35 in den Freiraum des Klemmorgans 37 ein. Im Schließzustand wird der Schlauch 23 zwischen den Klemmorganen 36 und 38 zusammengequetscht.

Von dem Klemmorgan 36 des Deckels 35 stehen zwei eindrückbare Rastelemente 39 nach entgegengesetzten Seiten hin ab. Diese Rastelemente rasten in Löchern 40 ein, die in den Wänden 37a und 37b des Ansatzes 37 vorgesehen sind. Wenn die Rastelemente 39 in die Rastlöcher 40 eintauchen, befindet sich der Deckel 35 im Schließzustand und der Schlauch 23 wird zwischen den Klemmorganen 36 und 38 zusammengequetscht. Zum Öffnen des

Deckels werden die Rastelemente 39 mit zwei Fingern von außen her eingedrückt, so daß die Verriegelung aufgehoben wird und der Deckel hochgeklappt werden kann. Auf diese Weise wird der Deckel durch einen Schnappverschluß im Schließzustand arretiert, so daß die Vortriebsscheibe 14 nicht mehr zugänglich ist und gleichzeitig der Schlauch 23 durch Quetschung sicher verschlossen ist.

Nach dem Hochklappen des Deckels 35 erfolgt durch Drehen der Vortriebsscheibe 14 der Ausstoß einer definierten Medikamentenmenge aus der Dosiervorrichtung. Nach Beendigung der Injektion wird der Deckel 35 lediglich heruntergeklappt und durch Einrasten des Schnappverschlusses geschlossen. Auf diese Weise wird ein Schutz gegen unbeabsichtigte Infusion geschaffen. Gleichzeitig gewährleistet der Deckel 35 einen Schutz gegen unbeabsichtigte Manipulationen an der Vortriebsscheibe.

Bei dem Ausführungsbeispiel der Fign. 8 und 9 ist das Betätigungsorgan 41 für die Klemmvorrichtung ebenfalls ein Deckel, der hier jedoch um eine vertikale Achse 42, die parallel zur Vortriebsrichtung des Vortriebsteils 14 verläuft, schwenkbar ist. Der Deckel 41 wird also in der Deckelebene verschwenkt. Von der der Achse 42 abgewandten Seite des Deckels 41 steht ein radialer Ansatz 43 ab, der das eine Klemmorgan 44 der Klemmvorrichtung trägt. Das andere Klemmorgan 45 befindet sich an einem radialen Ansatz 46, der von dem der Achse 42 abgewandten Ende des Gehäuses 10 absteht. In der Schließstellung des Deckels 41 werden die Klemmorgane 44 und 45 der Ansätze 43 und 46 gegeneinander gedrückt, wobei der zwischen diesen Klemmorganen 44 und 45 befindliche Schlauch 23 zusammengequetscht wird. Der Ansatz 43 weist eine Rastnase 47 auf, die im Schließzustand des Deckels einen Vorsprung 48 des Ansatzes 46 übergreift und so die Ansätze 43 und 46 gegeneinander verriegelt. Das Lösen der Verriegelung erfolgt durch Hochdrücken der flexiblen Rastnase 47.

Fig. 10 zeigt ein Ausführungsbeispiel, bei dem das Betätigungsorgan 41 ebenso wie bei dem vorhergehenden Ausführungsbeispiel ein Deckel ist, der in der Deckelebene um eine Achse 42 schwenkbar ist. Diese Achse 42 führt durch einen radialen Ansatz 48 des Gehäuses 10 hindurch. Über den Ansatz 48 verläuft der Schlauch 23, der durch ein Klemmorgan 45 dieses Ansatzes abgestützt ist. Das andere Klemmorgan 44 befindet sich in der Nähe der Achse 42 an dem Deckel 41. Auch hier gibt die Klemmvorrichtung 44,45 in der Öffnungsstellung des Deckels 41 den Schlauch 23 frei, während dieser Schlauch in der Schließstellung des Deckels 41 zwischen den Klemmorganen 44 und 45 zusammengequetscht wird. Die Verriegelungselemente, die den Deckel 41 in der Schließstellung verriegeln, befinden sich an den Ansätzen 46 und 47 an der der Achse 42 abgewandten Enden von Gehäuse 10 und Deckel 41.

0178371

ANSPRÜCHE

1. Dosiervorrichtung für Injektabilien, mit einem Behälter, dessen Volumen durch eine bewegbare Aus- stoßvorrichtung veränderbar ist, d a d u r c h  g e k e n n z e i c h n e t , daß der Behälter eine mit einer flexiblen Leitung (23) verbundene geschlossene verformbare Hülle (17) ist, die in einem starren Gehäuse (10) untergebracht ist, daß die Ausstoßvorrichtung aus einem am Gehäuse (10) abgestützten Vortriebsteil (14) besteht, das gegen die Hülle (17) drückt und relativ zu dem Gehäuse (10) bewegbar ist, und daß die Leitung (23) durch eine Öffnung (29) des Ge- häuses (10) hindurchragt.

2. Dosiervorrichtung nach Anspruch 1, dadurch gekennzeichnet, daß das Vortriebsteil (14) eine über Gewinde (13,15) mit dem Gehäuse (10) in Eingriff stehende Scheibe ist, die durch Drehen relativ zu dem Gehäuse bewegbar ist.

3. Dosiervorrichtung nach Anspruch 1 oder 2, dadurch gekennzeichnet, daß die Hülle (17) zwei parallele Wände (19,20) aufweist, zwischen denen sich eine umlaufende Faltenwand (21) erstreckt.

4. Dosiervorrichtung nach einem der Ansprüche 1 bis 3, dadurch gekennzeichnet, daß die Innenseite des Vortriebsteils (14) einen in Richtung auf die Hülle (17) vorstehenden koaxialen Vorsprung (24) aufweist, der von einem Ringkanal (25) zur Aufnahme des Randes der Hülle (17) umgeben ist.

5.  Dosiervorrichtung nach einem der Ansprüche 1 bis
    4, dadurch gekennzeichnet, daß die Unterseite der
    Hülle (17) durch ein starres Stützteil (26) abgestützt ist, dessen Profil im wesentlichen einem
    Negativ des Profils der Innenseite des Vortriebsteils (14) entspricht.

6.  Dosiervorrichtung nach einem der Ansprüche 1 bis
    5, dadurch gekennzeichnet, daß sich von der Öff-
    nung (29) ein Schlitz (28) bis zum deckelseitigen
    Rand des Gehäuses (10) erstreckt.

7.  Dosiervorrichtung nach Anspruch 2, dadurch gekennzeichnet, daß an dem Vortriebsteil (14) und dem
    Gehäuse (10) Markierungen (31,32) für die Anzeige
    der Drehstellung des Vortriebsteils (14) vorgesehen sind.

8.  Dosiervorrichtung nach einem der Ansprüche 1 bis
    7, dadurch gekennzeichnet, daß das Vortriebsteil
    (14) einen Schlitz (16) zum Einsetzen eines Drehwerkzeugs aufweist.

9.  Dosiervorrichtung nach einem der Ansprüche 1 bis
    8, dadurch gekennzeichnet, daß an dem Gehäuse (10)
    eine Klemmvorrichtung zum Zusammenquetschen eines
    Abschnitts der flexiblen Leitung (23) vorgesehen
    ist und daß die Klemmvorrichtung ein Betätigungsorgan (35;41) aufweist, das in einer Verriegelungsstellung das Verstellen des Vortriebsteils
    (14) verhindert und gleichzeitig die flexible
    Leitung (23) abquetscht und in einer Freigabestellung das Vortriebsteil (14) und die flexible
    Leitung (23) freigibt.

10. Dosiervorrichtung nach Anspruch 9, dadurch gekennzeichnet, daß das Betätigungsorgan (35;41) ein in der Verriegelungsstellung das Vortriebsteil (14) überdeckender Deckel des Gehäuses (10) ist.

11. Dosiervorrichtung nach Anspruch 10, dadurch gekennzeichnet, daß der Deckel um eine quer zur Bewegungsrichtung des Vortriebsteils (14) verlaufende Achse (34) des Gehäuses (10) herum aufklappbar ist und ein Klemmorgan (36) sowie ein an dem Gehäuse (10) einrastbares Verriegelungsorgan (39) trägt.

12. Dosiervorrichtung nach Anspruch 10, dadurch gekennzeichnet, daß der Deckel um eine parallel zur Bewegungsrichtung des Vortriebsteils (14) verlaufende Achse (42) in der Deckelebene schwenkbar ist und ein Klemmorgan (44) und ein an dem Gehäuse (10) einrastbares Verriegelungsorgan (47) trägt.

13. Dosiervorrichtung nach Anspruch 11 oder 12, dadurch gekennzeichnet, daß das Klemmorgan (44) und das Verriegelungsorgan (47) an dem Deckel einander benachbart angeordnet sind.

14. Dosiervorrichtung nach Anspruch 11 oder 12, dadurch gekennzeichnet, daß das Klemmorgan (44) und das Verriegelungsorgan (47) an entgegengesetzten Enden des Deckels angeordnet sind.

FIG.1

FIG.2

FIG.3

FIG. 4

FIG. 5

FIG. 6

0178371

FIG.7

**FIG. 8**

**FIG. 9**

**FIG. 10**